# EUROPÄISCHE PATENTSCHRIFT

(11) **EP 3 829 676 B1**
(45) Veröffentlichungstag und Bekanntmachung des Hinweises auf die Patenterteilung: **15.10.2025**
(21) Anmeldenummer: 19749290.3
(22) Anmeldetag: 30.07.2019
(51) Int. Cl.: A61M 1/16

(54) **VORRICHTUNG ZUR REGENERATION VON GEBRAUCHTER DIALYSELÖSUNG**
DEVICE FOR REGENERATION OF SPENT DIALYSATE
DISPOSITIF POUR LA RÉGÉNÉRATION DU DIALYSAT UTILISÉ

(30) Priorität: 31.07.2018 DE 102018118564
(43) Veröffentlichungstag der Anmeldung: 09.06.2021
(73) Patentinhaber: Fresenius Medical Care Deutschland GmbH, 61352 Bad Homburg (DE)
(72) Erfinder: KELLER, Torsten, 66606 St. Wendel (DE); MARTERSTOCK, Stefan Konrad, 97337 Dettelbach (DE)
(74) Vertreter: Herrmann, Uwe
(86) Internationale Anmeldenummer: PCT/EP2019/070415
(87) Internationale Veröffentlichungsnummer: WO 2020/025567

(56) Entgegenhaltungen:
- WO-A1-2014/128293
- WO-A1-2015/124716

## Beschreibung

Die vorliegende Erfindung betrifft eine Vorrichtung und ein Verfahren zur Herstellung einer Dialyselösung.

Aus dem Stand der Technik ist es bekannt, Dialysegeräte mit einer gebrauchsfertigen Dialyselösung z.B. aus einem Leitungssystem zu versorgen, das mit einer zentralen Einrichtung zur Herstellung der Dialyselösung verbunden ist und das im Allgemeinen ausgebildet ist, eine Mehrzahl von Dialysegeräten mit Dialyselösung zu versorgen. Alternativ dazu besteht die Möglichkeit, die Dialyselösung am Dialysegerät selbst, d.h. dezentral herzustellen. Dazu kann eine RO-Anlage (RO = Revers-Osmose) zur Herstellung von Reinstwasser vorgesehen sein, die Bestandteil des Dialysegerätes ist oder als eine gesonderte Einheit ausgeführt sein kann. Das Reinstwasser wird in dem Dialysegerät mit einem oder mehreren Konzentraten gemischt, um eine gebrauchsfertige Dialyselösung zur Behandlung des Patienten zu erhalten.

Der für diese Art der Herstellung der Dialyselösung erforderliche große Wasserbedarf führt dazu, dass bekannte Dialysegeräte eine zusätzliche RO- Anlage benötigen, so dass die Gesamtsysteme vergleichsweise groß und schwer sind und ein Transport der Geräte nur schwer möglich ist. Dieser Nachteil spielt eine besondere Rolle beispielsweise bei der Heimhämodialyse sowie bei portablen Geräten, die dem Patienten eine Mobilität während der Behandlung ermöglichen sollen.

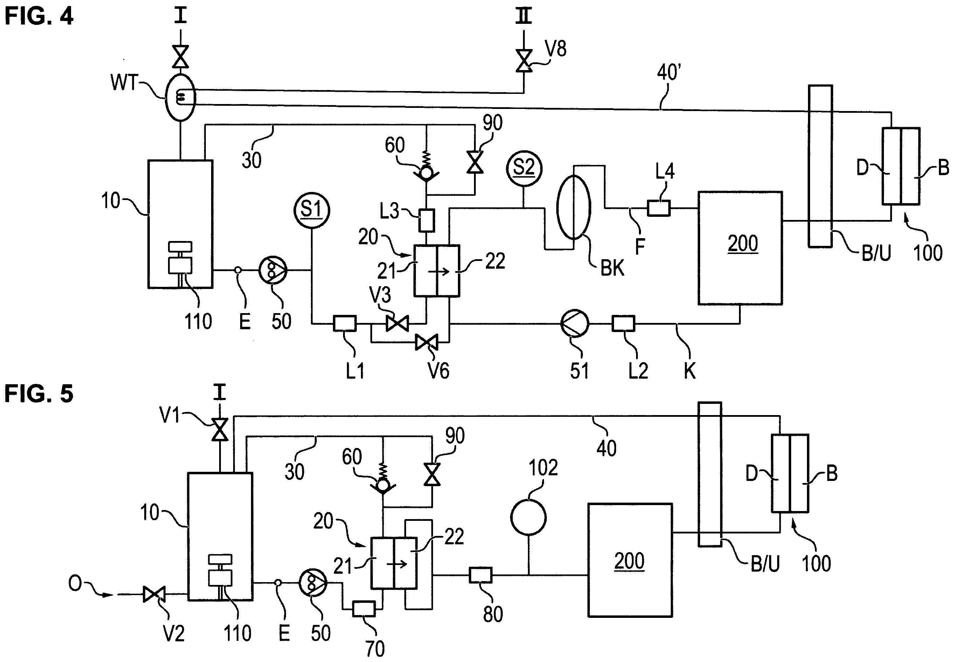

Figur 5 zeigt eine denkbare, nicht unter die vorliegende Erfindung fallende Möglichkeit einer Ausführung eines Dialysegerätes, bei dem der Wasserverbrauch dadurch reduziert wird, dass unter Verwendung gebrauchter Dialyseflüssigkeit neue, d.h. gebrauchsfertige Dialyseflüssigkeit gewonnen wird, was zu einer Verminderung des Wasserverbrauchs führt.

Mit dem Bezugszeichen I ist der Einlass für frisches Wasser in den Behälter 10 gekennzeichnet, wobei die Einlassleitung durch ein Ventil V1 absperrbar ist. Mit O ist der Ablauf aus dem Behälter 10 für gebrauchte Lösung gekennzeichnet, wobei die Ablaufleitung durch ein Ventil V2 absperrbar ist. Der Behälter 10 wird im Folgenden auch als Wassereingangskammer bezeichnet. Grundsätzlich kann ein Behälter 10 mit einem stationären Wasseranschluss oder auch ein beliebiger anderer Behälter, wie z.B. ein Beutel, wie z.B. ein Dialysatbeutel zum Einsatz kommen. Der Behälter 10 kann starre oder flexible Wandungen aufweisen.

Das Bezugszeichen 100 kennzeichnet einen Dialysator, der vorzugsweise eine Mehrzahl von Membranen, vorzugsweise ein Membranbündel aufweist, die auf einer Seite D von Dialysat und auf der anderen Seite B von Blut durchströmt werden. Die Dialyselösung, die den Dialysator 100 durchströmt hat und somit mit Verunreinigungen aus dem Blut beladen ist, wird als gebrauchte Dialyselösung oder gebrauchtes Dialysat bezeichnet. Das gebrauchte Dialysat, das vom Dialysator 100 durch die Leitung 40 zurück in die Wassereingangskammer 10 geleitet wird, wird von einer Pumpe 50 angesaugt und in einen vorgelagerten Kreislauf gepumpt, bei dem es sich um einen ersten Kreislauf handelt.

Dieser Kreislauf umfasst im Wesentlichen die Pumpe 50, den Behälter 10, die Primärseite des Filters 20, d.h. den Abschnitt vor der Filtermembran oder vor einem sonstigen Filtermedium, und das Druckbegrenzungsventil 60 einschließlich der diese Komponenten verbindenden Leitungen. Das Bezugszeichen 30 kennzeichnet die Rückführleitung von dem Druckbegrenzungsventil 60 zu dem Behälter 10.

Die Pumpe 50 fördert die gebrauchte Dialyselösung aus dem Behälter 10 zu der Primärseite 21 des Filters 20 und das Retentat über das Ventil 60 zurück in den Behälter 10. Dabei entsteht ein Druckabfall über dem Ventil 60 und somit auch ein Druck auf den Filter 20. Dieser Druck bzw. Druckabfall kann durch das Ventil 60 eingestellt und somit auf den idealen Arbeitspunkt des Filters 20 abgestimmt werden.

Der Druckgradient zwischen Primär- 21 und Sekundärseite 22 des Filters 20 führt dazu, dass eine Strömung durch die Filtermembran oder durch das sonstige Filtermedium stattfindet, wobei auf der Sekundärseite 22, d.h. nach dem Filtermedium das Filtrat in Form von Reinstwasser vorliegt. Es gelangt in die Mischeinrichtung 200, die als Mischkreislauf, Mischbehälter, Leitungsabschnitt etc. ausgeführt sein kann.

In dem Mischkreislauf oder Mischbehälter 200 etc. erfolgt die Mischung des Reinstwassers mit einem oder mehreren Konzentraten, wie z.B. mit einem basischen und einem sauren Konzentrat. Auch kann dort das Aufheizen bzw. Nachheizen der Dialyselösung erfolgen, damit dem Blut im Dialysator 100 möglichst keine Wärme durch die Dialyselösung entzogen wird. Ein zweiter Kreislauf umfasst somit die Sekundärseite 22 des Filters 20, die Mischeinrichtung 200, die Dialysatseite D des Dialysators 100 sowie ein diese Komponenten verbindendes Leitungssystem, wobei eine Leitung von der Dialysatseite D des Dialysators 100 zurück in den Wassereingangskammer 10 führt.

Wie aus Figur 5 ersichtlich, handelt sich bei dem ersten und zweiten Kreislauf um zwei geschlossene, kaskadierte Wasser- bzw. Flüssigkeitskreisläufe. Grundsätzlich können auch mehr als zwei dieser Kreisläufe vorhanden sein.

Der Filter 20 kann optional auf einer Graphen-Filtertechnik basieren, wodurch das Reinstwasser zugleich beispielsweise ohne gelösten Sauerstoff vom Filter abgeschieden werden kann. Dies spart weitere Komponenten in der Dialysemaschine, wie z.B. einen separaten Entgasungskreislauf.

Durch das "Überspülen" des Filters 20 auf dessen Primärseite 21 über das Ventil 60 kann eine hohe Performance erreicht werden und das Verschmutzen des Filters 20 kann verhindert bzw. hinausgezögert werden. Dadurch wird die Standzeit des Filters 20 erhöht. Zusätzlich kann der Filter 20 freigespült werden, indem das Ventil 90 das Druckbegrenzungsventil 60 kurzschließt. In diesem Fall gelangt die Flüssigkeit unter Umgehung des Ventils 60 in den Bypass, der das Ventil 90 enthält und von dort zurück in den Behälter.

Wie dies weiter aus Figur 5 hervorgeht, befindet sich in dem Behälter 10 ein Füllstandssensor 110. Meldet dieser, dass der Füllstand in den Behälter 10 unter einen Grenzwert gesunken ist, kann auf eine Leckage geschlossen werden, da sowohl der erste als auch der zweite Kreislauf geschlossen sind und somit der Füllstand gleich bleiben müsste. Die Ventile V1 und V2 sind im normalen Betrieb, d.h. wenn kein Wasserwechsel stattfindet, geschlossen.

Aus Figur 5 ergibt sich weiter das Vorhandensein einer Leitfähigkeitsmesszelle 70 nach, d.h. stromabwärts der Pumpe 50. Somit ist die durch den Kreislaufbetrieb bedingte Aufkonzentration der verunreinigten Flüssigkeit messbar und es lässt sich ableiten, wann das Wasser im Kreislauf komplett ausgetauscht werden sollte (Frischwassertaktung). Dieser Austausch kann durch Verwendung der Ventile V1 und V2 erfolgen und mit Hilfe des Levelsensors 110 in dem Behälter 10 volumetrisch bilanziert werden. Im normalen Dialysebetrieb sind die Ventile V1 und V2 geschlossen, wodurch der Levelsensor 10 wie ausgeführt auch als Leckageüberwachung eingesetzt werden kann. Sollte einer der beiden geschlossenen Kreisläufe Flüssigkeit verlieren, so ist dies anhand des Pegels in dem Behälter 10 detektierbar. Dies dient der Patientensicherheit.

Mit einer weiteren Leitfähigkeitsmesszelle 80 nach, d.h. stromabwärts des Filters 20 kann die Funktion des Filters 20 überwacht werden. Sobald eine relevante Schädigung der Filter-Membran oder eines sonstigen Filtermediums vorliegt, kommen leitfähige Ionen durch den Filter 20, die wiederum durch den Sensor 80 detektiert werden können. Optional kann mittels des stromabwärts des Filters 20 angeordneten Drucksensors 102 der Transmembrandruck über den Filter 20 überwacht werden, um eine Degradation bzw. Verlust der Performance des Filters feststellen zu können.

Da die beiden Kreisläufe geschlossen sind, sinkt der Energiebedarf für die Erwärmung des Dialysats deutlich. Als Konsequenz können kleinere Heizungen eingesetzt werden als dies bei aus dem Stand der Technik bekannten Geräten der Fall ist. Ein Wärmetauscher ist zwar noch optional für die Frischwassertaktung denkbar, aber kann aus Kostengründen auch entfallen. Wie oben ausgeführt, gelangt das auf der Sekundärseite 22 des Filters 20 anfallende Reinstwasser in den Mischteil 200 der Maschine und wird dort z.B. mit Bicarbonat und Säure angereicht, sodass eine gebrauchsfertige Dialysierflüssigkeit am Dialysator 100 zum Austausch mit dem Blut des Patienten zur Verfügung steht. Das Bezugszeichen B/U kennzeichnet die Bilanzierungseinheit und/oder eine Ultrafiltrationspumpe, die der gebrauchten Dialyseflüssigkeit ein der Verschreibung durch den Arzt entsprechendes Teilvolumen entzieht.

Durch den zweistufigen (kaskadierten) Filteransatz können auch weiterhin Giftstoffe, die größer als Wasser sind dem Patienten entnommen werden. Diese Stoffe, darunter auch Ionen wie Na und Cl aus der Dialysierflüssigkeit werden anschließend in dem Behälter 10 aufkonzentriert und im Zuge der Frischwassertaktung über den Ausfluss O verworfen. Die Ultrafiltration geschieht nach wie vor z.B. über eine UF-Pumpe in der Einheit B/U, welche direkt in den Abfluss fördert. Da es sich um geschlossene Kreisläufe handelt, können die Vorteile der volumetrischen Bilanzierung, wie dies heutzutage bspw. mit Hilfe einer Bilanzkammer erfolgt, gewahrt werden.

Durch die Eigenschaften des Filters 20, für Gase undurchlässig zu sein, entfällt in diesem Aufbau der Bedarf für zusätzliche Entgasungsmaßnahmen/-vorrichtungen. Alternativ kann bei Bedarf, je nach eingesetztem Filter, aber auch eine Entgasungsdrossel/-vorrichtung in den vorgelagerten, d.h. in den ersten Kreislauf eingebracht werden, wie dies durch das Bezugszeichen E in Figur 5 gezeigt ist. Als Luftabscheidekammer kann aber auch eine separate Kammer dienen, die bspw. zwischen 20 und 60 in Figur 5 angeordnet werden kann.

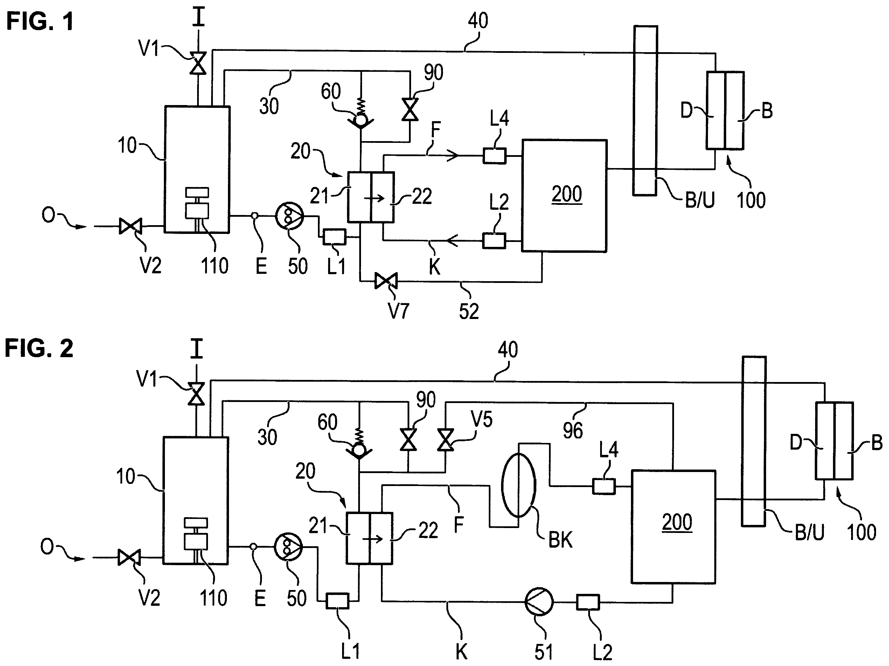

An dieser Stelle wird darauf hingewiesen, dass sämtliche der zu Figur 5 erläuterten Merkmale einzeln oder auch in Kombination Gegenstand der vorliegenden Erfindung sein können. Weiter wird darauf hingewiesen, dass gleiche Bezugszeichen in Figur 5 sowie in den Figuren 1 bis 4 gleiche oder funktionsgleiche Elemente kennzeichnen.

Die Anordnung gemäß Figur 5 ist mit dem Nachteil behaftet, dass im Laufe der Zeit eine Aufkonzentrierung der in dem Behälter 10 befindlichen Lösung stattfindet. Somit liegt auch an der Primärseite 21 des Filters 20 eine mit der Dauer der Behandlung zunehmend stärker aufkonzentrierte Lösung vor. Der durch diese Lösung erzeugte osmotische Druck erschwert die Filtration in dem Filter 20, da er der Bildung von Filtrat entgegenwirkt. Mit einer zunehmenden Aufkonzentrierung auf der Primärseite 21 wird daher ein zunehmender durch die Pumpe 50 erzeugter Druck notwendig, um eine ausreichende Menge an Reinstwasser auf der Sekundärseite 22 des Filters 20 zu erhalten. Vorrichtungen, die zum Stand der Technik gehören, sind in den Dokumenten WO 2014 128293 und WO 2015/124716 beschrieben.

Der vorliegenden Erfindung liegt die Aufgabe zugrunde, eine Vorrichtung bereitzustellen, mit der eine effiziente Reinstwassergewinnung möglich ist.

Diese Aufgabe wird durch eine Vorrichtung mit den Merkmalen des Anspruchs 1 gelöst.

Danach ist vorgesehen, dass die Vorrichtung zur Herstellung einer Dialyselösung einen ersten Kreislauf und einen zweiten Kreislauf aufweist, wobei der erste Kreislauf einen Behälter zur Aufnahme der gebrauchten Dialyselösung oder von Frischwasser oder einer sonstigen Flüssigkeit, die Primärseite eines dem Behälter nachgeschalteten Filters sowie eine Rückführleitung von der Primärseite des Filters in den Behälter aufweist, wobei der Filter ausgebildet ist, aus der gebrauchten Dialyselösung oder aus Frischwasser oder aus einer sonstigen Flüssigkeit gereinigtes Wasser herzustellen, und wobei der zweite Kreislauf die Sekundärseite des Filters, die Dialysatseite eines Dialysators, ein Reservoir, eine sich von dem Reservoir zu der Sekundärseite des Filters führende Leitung, die im Folgenden auch als Konzentratleitung bezeichnet wird, mittels derer Dialysat oder ein Dialysatkonzentrat der Sekundärseite des Filters zuführbar ist, sowie eine Filtratleitung aufweist, die von der Sekundärseite des Filters wegführt.

Nach einer Ausführungsform ist vorgesehen, dass das Reservoir Konzentrate wie ein Bicarbonatkonzentrat oder ein saures Konzentrat oder eine gebrauchsfertige Dialyselösung oder eine Mischung aus einem basischen Konzentrat, insbesondere einem Bicarbonatkonzentrat, und einem sauren Konzentrat aufnimmt.

Im Gegensatz zu der aus Figur 5 ersichtlichen Anordnung, wird der Sekundärseite des Filters eine Lösung entweder in Form einer gebrauchsfertigen Dialyselösung oder bevorzugt in der Form eines flüssigen Konzentrats zugeführt. Dies hat den Vorteil, dass der osmotische Druckgradient über die Filtermembran entsprechend abnimmt und somit eine effizientere Herstellung von Filtrat möglich ist.

Vorzugsweise handelt es sich bei dem Konzentrat, das der Sekundärseite des Filters zugeführt wird, um ein Konzentrat, das zur Herstellung der Dialyselösung benötigt wird und besonders bevorzugt um ein bicarbonathaltiges Konzentrat. Besonders bevorzugt ist es, wenn das Konzentrat außer Wasser und gegebenenfalls Kochsalz nur eine einzige leitfähige Komponente, wie z.B. Bicarbonat enthält.

Der Filter hat die Aufgabe, aus der in dem Behälter befindlichen Lösung gereinigtes Wasser herzustellen, d.h. Wasser, dessen Gehalt bzw. Konzentration an Verunreinigungen und sonstigen Inhaltsstoffen, wie Ionen oder Molekülen geringer ist als in der Lösung, die dem Filter zugeführt wird. Vorzugsweise ist der Filter ausgebildet, Reinstwasser herzustellen, worunter im Rahmen der vorliegenden Erfindung Wasser verstanden wird, das geeignet ist, zur Herstellung einer gebrauchsfertigen Dialyselösung verwendet zu werden. Der Filter kann ein oder mehrstufig ausgeführt sein, wobei die mehreren Stufen nacheinander von Lösung durchströmt werden. Auch ist der Einsatz mehrerer in Reihe geschalteter Filter denkbar, um den gewünschten Reinheitsgrad des Wassers zu erhalten. Bei dem Filter handelt es sich bevorzugt um ein Hohlfaser- oder Wickelmodul, wie es für RO- (Reverse Osmose) oder FO (Forward Osmose) Vorgänge verwendet wird.

Vorzugsweise handelt es sich bei dem genannten Filter um einen Graphen-Filter. Darunter wird ein Filter verstanden, der als Filtermaterial Graphen oder ein Graphen-Derivat, wie z.B. Graphenoxid enthält oder dessen Filtermaterial aus diesen Stoffen besteht oder diese enthält. Graphen bzw. Graphenoxid ist gasdicht, aber zugleich wasserdurchlässig, was im Rahmen der vorliegenden Erfindung den Vorteil mit sich bringt, dass ein Gaseintrag von dem ersten in den zweiten Kreislauf und damit in die gebrauchsfertige Dialyselösung nicht erfolgt. Die vorliegende Erfindung ist aber nicht auf diese Filter beschränkt, sondern um fasst auch andere Filter, die vorzugsweise gasundurchlässig sind, jedoch Flüssigkeit passieren lassen. Sollte der verwendete Filter die Eigenschaft der Gasundurchlässigkeit nicht aufweisen so kann bspw. durch eine Entgasungsdrossel Luft primärseitig abgeschieden werden, welche dann über ein Ventil (vorzugsweise ein druckbegrenzendes Ventil) zurück in den Behälter gefördert und somit entfernt werden kann. Auch andere Entgasungsvorgänge sind möglich.

Der Filter weist vorzugsweise vier Anschlüsse auf, von denen zwei primärseitig und zwei sekundärseitig vorgesehen sind.

In einer bevorzugten Ausgestaltung der Erfindung ist eine Rückführleitung von der Dialysatseite des Dialysators in den Behälter vorhanden, durch die gebrauchte Dialyselösung dem Behälter zugeführt wird. Alternativ dazu kann vorgesehen sein, dass eine Rückführleitung von der Dialysatseite des Dialysators in einen Ablauf vorhanden ist, d.h. die gebrauchte Dialyselösung verworfen wird.

Die Filtratleitung, die von der Sekundärseite des Filters wegführt, kann die Sekundärseite des Filters mit einer Bilanzkammer der Vorrichtung verbinden. In diesem Fall wird das durch den Filter gewonnene Filtrat, vorzugsweise Reinstwasser z.B. mit dem Konzentrat gemischt und diese Mischung wird der Bilanzkammer des Dialysegerätes zugeführt. Handelt es sich bei dem Konzentrat um eine BicarbonatLösung, wird somit der Bilanzkammer eine durch das Reinstwasser verdünnte Bicarbonatlösung zugeführt. In einem weiteren Schritt kann dieser Mischung sodann das saure Konzentrat zugeführt werden oder die verdünnte Bicarbonatlösung etc. kann in einen Behälter, wie z.B. in das Reservoir eingefüllt werden, in dem bereits eine definierte Menge an weiterem Konzentrat oder sonstigen für die gebrauchsfertige Dialyselösung notwendigen Komponenten vorliegen.

In einer weiteren Ausführungsform kann vorgesehen sein, dass der Filtratseite des Filters direkt ein Konzentrat bestehend aus dem sauren und dem basischen Konzentrat zugeführt wird. Die Konzentratlösung dieser Ausführungsform bewirkt eine besonders günstige Filtrationsleistung des Filters. Das Dialysatkonzentrat umfasst gemäß dieser Ausführungsform sowohl ein basisches, insbesondere eine Bicarbonatkonzentrat, als auch ein saueres Konzentrat.

Auch ist es denkbar, dass die Filtratleitung in Fluidverbindung mit dem Reservoir steht, so dass z.B. das durch den Filter gewonnene Filtrat, vorzugsweise Reinstwasser z.B. mit dem Konzentrat gemischt und diese Mischung dem Reservoir des Dialysegerätes zugeführt wird. Vorzugsweise ist das Reservoir bzw. die Mischkammer der Bilanzkammer nachgeschaltet.

Weiterhin ist es denkbar, dass in dem Reservoir gebrauchsfertige Dialyselösung oder ein Konzentrat, insbesondere ein Bicarbonat-Konzentrat, vorliegt, das zur Herstellung einer gebrauchsfertigen Dialyselösung verwendet wird.

Vorzugsweise handelt es sich bei dem Reservoir um eine Dialysatmischeinrichtung, in der die gebrauchsfertige Dialyselösung gemischt wird und von dort der Dialysatseite des Dialysators zugeführt wird.

Vorzugsweise befindet sich in der Konzentratleitung eine Pumpe, die ausgebildet ist, der Sekundärseite des Filters ein definiertes Volumen oder einen definierten Volumenstrom zuzuführen. Bei der Pumpe kann es sich beispielsweise um eine volumetrische Pumpe bzw. um eine Exzentermembranpumpe handeln. Auf diese Weise ist es möglich, einen rein volumengesteuerten Mischprozess zu realisieren, der beispielsweise durch eine unabhängige Leitfähigkeitsmessung überwacht werden kann.

Weiterhin kann vorgesehen sein, dass in dem ersten Kreislauf, vorzugsweise stromaufwärts der Primärseite des Filters eine Pumpe angeordnet ist, um eine Strömung von Flüssigkeit in dem ersten Kreislauf zu bewirken. Diese Pumpe fördert die in dem Behälter befindliche Flüssigkeit zu der Primärseite des Filters.

Alternativ oder zusätzlich kann vorzugsweise stromabwärts des Filters in dem ersten Kreislauf eine Druckbegrenzungseinrichtung vorgesehen sein, mittels derer Druck auf der Primärseite des Filters einstellbar ist.

Zur Überwachung bzw. Steuerung des mittels der Vorrichtung durchgeführten Prozesses können in dem ersten Kreislauf ein oder mehrere Sensoren, vorzugsweise eine oder mehrere Leitfähigkeitsmesszellen angeordnet sein, die stromaufwärts und stromabwärts der Primärseite des Filters angeordnet sind. Alternativ oder zusätzlich können in dem zweiten Kreislauf in der Konzentratleitung und/oder in der Filtratleitung ein oder mehrere Sensoren, vorzugsweise eine oder mehrere Leitfähigkeitsmesszellen angeordnet sein.

Weiterhin kann vorgesehen sein, dass stromabwärts der Sekundärseite des Filters und/oder stromaufwärts der Primärseite des Filters eine oder mehrere Druckmesseinrichtungen angeordnet sind. Auf diese Weise lässt sich eine Aussage über die Druckverhältnisse in dem jeweiligen Kreislauf sowie auch über den transmembranen Druck über die Filtermembran erzielen.

Weiterhin kann die Vorrichtung eine Ultrafiltratpumpe zur Abfuhr von Dialyselösung, vorzugsweise aus der Rückführleitung vom Dialysator zu dem Behälter und/oder eine Bilanzierkammer zum bilanzierten Zu- und Abführen von Dialyselösung zu dem und von dem Dialysator aufweisen.

Der erste und/oder der zweite Kreislauf können geschlossen oder offen sein. Unter "offen" ist zu verstehen, dass kein Kreislauf im engeren Sinne vorliegt, sondern ein Fluidsystem, das an zumindest einer Stelle eine Ableitung aufweist, mittels derer eine Flüssigkeit aus dem Kreislauf abgeleitet und verworfen werden kann.

Der Behälter kann als Beutel, insbesondere als flexibler Beutel, weiter insbesondere als Einmalartikel ausgebildet sein.

Die beanspruchte Vorrichtung kann ein Dialysegerät sein oder einen Teil eines Dialysegerätes oder eine Vorrichtung zur Anmischung von Dialysierlösung bilden.

Die vorliegende Erfindung betrifft des Weiteren ein Verfahren zur Herstellung einer Dialyselösung mit einer Vorrichtung gemäß der Erfindung, wobei die Lösung aus dem Behälter der Primärseite des Filters zugeführt wird und das Retentat in den Behälter zurückgeführt wird, wobei der Sekundärseite des Filters eine Dialyselösung oder ein Dialysatkonzentrat zugeführt wird, das mit dem Permeat des Filters auf dessen Sekundärseite gemischt wird.

Vorzugsweise wird die der Sekundärseite des Filters zugeführte Dialyselösung oder ein Dialysatkonzentrat aus dem genannten Reservoir abgeführt und der Sekundärseite des Filters zugeleitet.

**In** der Konzentratleitung kann eine Pumpe angeordnet sein, die der Sekundärseite des Filters ein definiertes Volumen oder einen definierten Volumenstrom an Dialyselösung oder an Dialysatkonzentrat zuführt.

Weiterhin kann vorgesehen sein, dass das auf der Sekundärseite des Filters anfallende Permeat zusammen mit der zugeführten Dialyselösung bzw. dem Dialysatkonzentrat in das Reservoir und/oder in eine Bilanzkammer geleitet wird.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: ein schematisches Flussbild einer Vorrichtung gemäß der Erfindung in einer ersten Ausführungsform der Erfindung,
- Figur 2:: ein schematisches Flussbild einer Vorrichtung gemäß der Erfindung in einer zweiten Ausführungsform der Erfindung,
- Figur 3:: ein schematisches Flussbild einer Vorrichtung gemäß der Erfindung in einer dritten Ausführungsform der Erfindung,
- Figur 4:: ein schematisches Flussbild einer Vorrichtung gemäß der Erfindung in einer vierten Ausführungsform der Erfindung,
- Figur 5:: ein schematisches Flussbild einer weiteren Vorrichtung, die nicht Gegenstand der Erfindung ist.

Wie oben ausgeführt, zeigt Figur 5 zeigt eine Variante, die nicht Gegenstand der Erfindung ist. Gleiche oder funktionsgleiche Elemente gemäß Figur 5 weisen dieselben Bezugszeichen auf wie in Figur 1 bis 4, so dass hinsichtlich des Aufbaus der in den Figuren 1 bis 4 dargestellten Vorrichtungen auch auf Figur 5 verwiesen wird.

Wie dies aus Figur 1 hervorgeht, unterscheidet sich die in Figur 1 dargestellte Vorrichtung gegenüber der Anordnung nach Figur 5 im Wesentlichen dadurch, dass von dem Reservoir 200 eine Leitung K zu der Sekundärseite 22 des Filters verläuft, durch die die gebrauchsfertige Dialyselösung strömt. In dieser Leitung K befindet sich der Leitfähigkeitssensor L2.

Ablaufseitig führt von der Sekundärseite 22 des Filters 20 eine Filtratleitung F zu dem Reservoir 200. Diese Leitung führt eine Mischung des über die Leitung K zugeführten Dialysats mit der mittels des Filters 20 z.B. durch RO gewonnenen Reinstwassers. In der Leitung F befindet sich der Leitfähigkeitssensor L4.

Darüber hinaus ist eine Leitung 52 vorgesehen, die durch das Ventil V7 verschließbar ist und durch die das Reservoir 200 mit der Leitung in Fluidverbindung steht, durch die Flüssigkeit aus dem Behälter der Primärseite 21 des Filters 20 zugeführt wird. In der letztgenannten Leitung befindet sich die Pumpe 50 sowie der Leitfähigkeitssensor L1. Dementsprechend steht bei geöffnetem Ventil das Reservoir 200 in Fluidverbindung mit der Primärseite 21 des Filters 20.

Bei einer Vorrichtung nach Fig. 1 wird der Stoffaustausch bzw. der Volumenübergang über den Filter durch die Leitfähigkeitssensoren L2 und L4 gemessen und sichergestellt. Figur 2 zeigt eine Ausführungsform, bei der durch die Leitung K ein Konzentrat oder eine Konzentratmischung, insbesondere ein Bicarbonatkonzentrat gefördert wird. Dazu dient die in der Leitung K befindliche Pumpe 51. Auf die Sekundärseite 22 des Filters 20 wird somit eine aufkonzentrierte Lösung gefördert, wobei deren Leitfähigkeit durch den Leitfähigkeitssensor L2 bestimmt wird. Mittels der Pumpe 51 wird ein bestimmtes, d.h. definiertes Volumen dieses Konzentrats bzw. der Mischung gefördert. Dieses Konzentrat bzw. diese Mischung wird in dem Filter 20 mit Reinstwasser gemischt, das das Konzentrat bzw. die Konzentratmischung auf der Sekundärseite verdünnt.

Dies Mischung wird der Bilanzkammer BK zugeführt, die mit vier Ventilen ausgeführt ist und die zwei durch eine bewegbare Trennwand getrennte Kammern aufweist, von denen jede ein Zulauf- und ein Ablaufventil aufweist.

Somit erhält die Bilanzkammer BK das durch Reinstwasser verdünntes Konzentrat oder Konzentratgemisch. Somit ergibt sich ein rein volumengesteuerter Mischprozess, der durch eine unabhängige Leitfähigkeitsmessung (Leitfähigkeitssensor L4) überwacht werden kann. Zur Leitfähigkeit trägt (im Gegensatz zu der Ausführung gemäß Figur 1) nur das Konzentrat als einer Komponente der gebrausfertigen Dialyselösung bei. Somit kann ein Erwartungswert für die Leitfähigkeit errechnet werden. Die Regelung auf einen Erwartungswert einer Leitfähigkeit lässt sich einfach durchführen.

Denkbar ist es auch, eine Regelung bzw. einen Regelkreis vorzusehen, dessen Sollgröße das der Bilanzkammer zugeführte Volumen an verdünntem Konzentrat/Konzentratmischung ist, wobei der Istwert durch die Leitfähigkeitsmessung bereitgestellt wird. Entscheidend ist hierfür das konkrete Rückhaltevermögen des Filters. Bei Kenntnis der Leitfähigkeit in der Leitung K sowie bei Kenntnis der Leitfähigkeit in der Leitung F kann ermittelt werden, um welchen Betrag das von der Pumpe geförderte Volumen aufgrund des Übertritts von Reinstwasser zugenommen hat, d.h. welches Volumen der Bilanzkammer bzw. dem dieser nachgeschalteten Reservoir 200 zugeführt wird.

Wie dies weiter aus Figur 2 hervorgeht, steht die Primärseite 21 des Filters 20 bzw. die von dieser abführenden Leitungen über die Leitung 96, in der sich das Ventil V5 befindet, mit dem Reservoir 200 in Fluidverbindung.

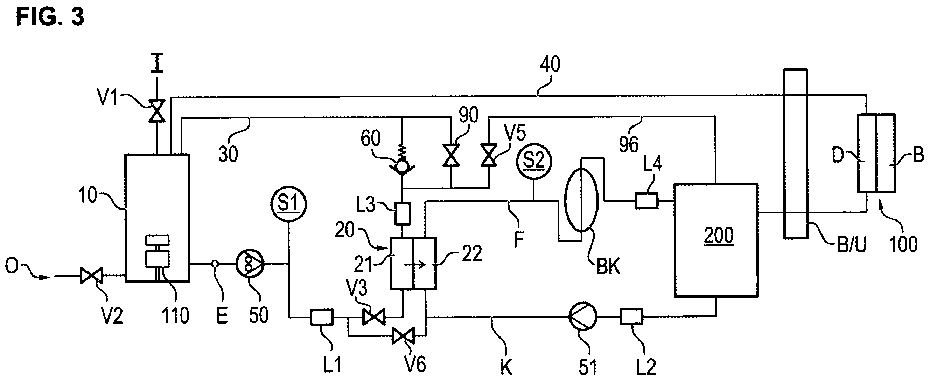

Die Ausführungsform gemäß Figur 3 unterscheidet sich von der Variante gemäß Figur 2 durch insgesamt vier Leitfähigkeitssensoren L1 - L4, von denen zwei stromaufwärts und stromabwärts der Primärseite 21 des Filters und zwei in der Konzentratleitung K und in der Filtratleitung F angeordnet sind. Bei Kenntnis der Ein- und Ausgangsleitfähigkeit kann der Prozess zusätzlich plausibilisiert, gesteuert und überwacht werden.

Des Weiteren sind Druckmesssensoren S1 und S2 vorgesehen, die der Überwachung des Transmembrandrucks über die Membran des Filters 20 dienen. Der Sensor S1 befindet sich zulaufseitig auf der Primärseite des Filters 20 und der Sensor S2 in der Filtratleitung F. Die Werte der Sensoren können verwendet werden, um ein Degradation des Filters F z.B. durch Scaling erkennen zu können und Spülzyklen / Regenerationszyklen des Filters planen und durchführen zu können.

Mittels des Sensors S2 kann zudem erkannt werden, ob ausreichend Reinstwasser über den Filter 20 auf dessen Sekundärseite 22 geströmt ist und die Bilanzkammer voll ist, so dass das gewünschte Mischungsverhältnis erreicht ist und die Bilanzkammer umgeschaltet bzw. getaktet werden kann. Sodann kann ein neuer "Anmischzyklus" durch die Pumpe 51 gestartet werden.

Alternativ kann die Auswertung des Drehmomentes bzw. des Motorstromes der Pumpe 51 für die Erreichung des gewünschten Mischungsverhältnisses herangezogen werden.

Der Wert des Leitfähigkeitssensors L1 kann verwendet werden, um die Frischwassertaktung zu steuern, d.h. zu erkennen, wann das Retentat in dem Behälter 10 durch Frischwasser zu ersetzen ist.

Von der Erfindung ist grundsätzlich nicht nur der Einsatz eines Filters 20 umfasst, sondern auch der Einsatz mehrerer hintereinander geschalteter, d.h. kaskadierter Filter umfasst. Dies entlastet die Pumpe 50, setzt aber das Vorhandensein mehrerer Pumpen voraus.

Die Kammer 10 kann als starrer Behälter oder als Beutel ausgeführt sein.

Der Filter 20 kann durch Öffnen z.B. der Ventile V3 und 90 gespült und damit gereinigt werden.

Über das Ventil V5 oder V6 kann alternativ die Sekundärseite initial mit Frischwasser gefüllt werden. Vorteilhaft ist jedoch eine Befüllung mit Frischwasser über den Filter 20 und die Bilanzkammer BK.

Wie dies aus Figur 4 hervorgeht, kann das Verfahren auch ohne die Rückführung von gebrauchtem Dialysat in den Behälter 10 durchgeführt werden. In diesem Fall wird das gebrauchte Dialysat mittels der Leitung 40' verworfen. Über den Wärmetauscher WT erfolgt eine Vorwärmung des dem Behälter 10 zugeführten Wassers mittels der gebrauchten Dialyselösung.

## Patentansprüche

1. Vorrichtung zur Herstellung einer Dialyselösung, wobei die Vorrichtung einen ersten Kreislauf und einen zweiten Kreislauf aufweist, wobei der erste Kreislauf einen Behälter (10) zur Aufnahme der gebrauchten Dialyselösung oder von Frischwasser oder einer sonstigen Flüssigkeit, die Primärseite (21) eines dem Behälter (10) nachgeschalteten Filters (20) sowie eine Rückführleitung (30) von der Primärseite (21) des Filters (20) in den Behälter (10) aufweist, wobei der Filter (20) ausgebildet ist, aus der gebrauchten Dialyselösung oder aus Frischwasser oder aus einer sonstigen Flüssigkeit gereinigtes Wasser herzustellen, und wobei der zweite Kreislauf die Sekundärseite (22) des Filters (20), die Dialysatseite eines Dialysators (100), ein Reservoir (200), eine sich von dem Reservoir (200) zu der Sekundärseite (22) des Filters führende Leitung (K), mittels derer Dialysat oder ein Dialysatkonzentrat der Sekundärseite (22) des Filters (20) zuführbar ist, sowie eine Filtratleitung (F) aufweist, die von der Sekundärseite (22) des Filters (20) wegführt.

2. Vorrichtung nach Anspruch 1, **dadurch gekennzeichnet, dass** eine Rückführleitung (40) von der Dialysatseite des Dialysators (100) in den Behälter (10) vorhanden ist oder dass eine Leitung (41') von der Dialysatseite des Dialysators (100) in einen Ablauf vorhanden ist.

3. Vorrichtung nach Anspruch 1 oder 2, **dadurch gekennzeichnet, dass** es sich bei dem Filter (20) um einen Graphen-Filter handelt.

4. Vorrichtung nach einem der vorhergehenden Ansprüche, **dadurch gekennzeichnet, dass** die Filtratleitung (F) von der Sekundärseite (22) des Filters (20) zu einer Bilanzkammer (BK) der Vorrichtung und/oder zu dem Reservoir (200) führt.

5. Vorrichtung nach einem der vorhergehenden Ansprüche, **dadurch gekennzeichnet, dass** in dem Reservoir (200) gebrauchsfertige Dialyselösung oder ein Konzentrat, insbesondere ein Bicarbonat-Konzentrat, vorliegt, das zur Herstellung einer gebrauchsfertigen Dialyselösung verwendet wird.

6. Vorrichtung nach einem der vorhergehenden Ansprüche, **dadurch gekennzeichnet, dass** es sich bei dem Reservoir (200) um eine Dialysatmischeinrichtung (200) handelt.

7. Vorrichtung nach einem der vorhergehenden Ansprüche, **dadurch gekennzeichnet, dass** in der Leitung (K) eine Pumpe (51) angeordnet ist, die ausgebildet ist, der Sekundärseite (22) des Filters (20) ein definiertes Volumen oder einen definierten Volumenstrom zuzuführen.

8. Vorrichtung nach einem der vorhergehenden Ansprüche, **dadurch gekennzeichnet, dass** in dem ersten Kreislauf, vorzugsweise stromaufwärts der Primärseite (21) des Filters (20) eine Pumpe (50) angeordnet ist, um eine Strömung von Flüssigkeit in dem ersten Kreislauf zu bewirken und/oder dass vorzugsweise stromabwärts des Filters (20) in dem ersten Kreislauf eine Druckbegrenzungseinrichtung (60) vorgesehen ist, mittels derer Druck auf der Primärseite (21) des Filters (20) einstellbar ist.

9. Vorrichtung nach einem der vorhergehenden Ansprüche, **dadurch gekennzeichnet, dass** in dem ersten Kreislauf ein oder mehrere Sensoren, vorzugsweise eine oder mehrere Leitfähigkeitsmesszellen (L1, L3) angeordnet sind, die stromaufwärts und stromabwärts der Primärseite (21) des Filters (20) angeordnet sind und/oder **dadurch gekennzeichnet, dass** in dem zweiten Kreislauf in der Leitung (K) und/oder in der Filtratleitung (F) ein oder mehrere Sensoren, vorzugsweise eine oder mehrere Leitfähigkeitsmesszellen (L2, L4) angeordnet sind.

10. Vorrichtung nach einem der vorhergehenden Ansprüche, **dadurch gekennzeichnet, dass** stromabwärts der Sekundärseite (22) des Filters (20) und/oder stromaufwärts der Primärseite (21) des Filters (20) eine oder mehrere eine Druckmesseinrichtungen (S1, S2) angeordnet sind.

11. Vorrichtung nach einem der vorhergehenden Ansprüche, **dadurch gekennzeichnet, dass** die Vorrichtung eine Ultrafiltratpumpe zur Abfuhr von Dialyselösung, vorzugsweise aus der Rückführleitung (40) vom Dialysator (100) zu dem Behälter (10) und/oder eine Bilanzierkammer (B) zum bilanzierten Zu- und Abführen von Dialyselösung zu dem und von dem Dialysator (100) aufweist.

12. Vorrichtung nach einem der vorhergehenden Ansprüche, **dadurch gekennzeichnet, dass** der Filter (20) gasundurchlässig ist und/oder dass der zweite Kreislauf keine Entgasungseinrichtung aufweist.

13. Vorrichtung nach einem der vorhergehenden Ansprüche, **dadurch gekennzeichnet, dass** der erste und/oder der zweite Kreislauf geschlossen oder offen sind.

14. Vorrichtung nach einem der vorhergehenden Ansprüche, **dadurch gekennzeichnet, dass** der Behälter (10) als starres Gefäß oder als Beutel, insbesondere als flexibler Beutel, weiter insbesondere als Einmalartikel (Disposable) ausgebildet ist und/oder **dadurch gekennzeichnet, dass** die Vorrichtung ein Dialysegerät oder einen Teil eines Dialysegerätes bildet.

15. Verfahren zur Herstellung einer Dialyselösung mit einer Vorrichtung nach einem der Ansprüche 1 bis 14, **dadurch gekennzeichnet, dass** die Lösung aus dem Behälter (10) der Primärseite (21) des Filters (20) zugeführt wird und das Retentat in den Behälter (10) zurückgeführt wird, wobei der Sekundärseite (22) des Filters (20) eine Dialyselösung oder ein Dialysatkonzentrat zugeführt wird, das mit dem Permeat des Filters (20) auf dessen Sekundärseite (22) gemischt wird.

16. Verfahren nach Anspruch 15, **dadurch gekennzeichnet, dass** das Permeat zusammen mit der Dialyselösung oder dem Dialysatkonzentrat einem Reservoir (200) zugeführt wird, in dem eine gebrauchsfertige Dialyselösung hergestellt wird, die einem Dialysator (100) zugeführt wird,

17. Verfahren nach Anspruch 16, **dadurch gekennzeichnet, dass** der Mischbereich (200) durch das Reservoir (200) gebildet wird.

18. Verfahren nach einem der Ansprüche 15 bis 17, **dadurch gekennzeichnet, dass** das Dialysatkonzentrat außer Wasser nur eine einzige leitfähige Komponente enthält.

19. Verfahren nach einem der Ansprüche 15 bis 18, **dadurch gekennzeichnet, dass** der Leitung (K) eine Pumpe (51) angeordnet ist, die der Sekundärseite (22) des Filters (20) ein definiertes Volumen oder einen definierten Volumenstrom an Dialyselösung oder an Dialysatkonzentrat zuführt.

20. Verfahren nach einem der Ansprüche 15 bis 19, **dadurch gekennzeichnet, dass** das auf der Sekundärseite (22) des Filters (20) anfallende Permeat zusammen mit der zugeführten Dialyselösung bzw. dem Dialysatkonzentrat in eine Bilanzkammer (BK) geleitet wird.

21. Verfahren nach einem der Ansprüche 15 bis 20, **dadurch gekennzeichnet, dass** das auf der Sekundärseite (22) des Filters (20) anfallende Permeat zusammen mit der zugeführten Dialyselösung bzw. dem Dialysatkonzentrat in das Reservoir (200) bzw. in den Mischbereich (200) geleitet wird.

## Claims

1. Apparatus for preparing a dialysis solution, wherein the apparatus has a first circuit and a second circuit, wherein the first circuit comprises a container (10) for receiving the consumed dialysis solution or fresh water or another fluid, the primary side (21) of a filter (20) connected downstream of the container (10), and a return line (30) from the primary side (21) of the filter (20) into the container (10), wherein the filter (20) is configured to prepare purified water from the consumed dialysis solution or from fresh water or from another fluid, and wherein the second circuit comprises the secondary side (22) of the filter (20), the dialyzate side of a dialyzer (100), a reservoir (200), a line (K) that leads from the reservoir (200) to the secondary side (22) of the filter and by means of which dialyzate or a dialyzate concentrate can be supplied to the secondary side (22) of the filter (20), and a filtrate line (F) that leads away from the secondary side (22) of the filter (20).

2. Apparatus in accordance with claim 1, **characterized in that** a return line (40) from the dialyzate side of the dialyzer (100) into the container (10) is present; or **in that** a line (41') is present from the dialyzate side of the dialyzer (100) into a drain.

3. Apparatus in accordance with claim 1 or claim 2, **characterized in that** the filter (20) is a graphene filter.

4. Apparatus in accordance with any one of the preceding claims, **characterized in that** the filtrate line (F) leads from the secondary side (22) of the filter (20) to a balancing chamber (BK) of the apparatus and/or to the reservoir (200).

5. Apparatus in accordance with any one of the preceding claims, **characterized in that** ready-to-use dialysis solution or a concentrate, in particular a bicarbonate concentrate, is present in the reservoir (200) that is used to prepare a ready-to-use dialysis solution.

6. Apparatus in accordance with any one of the preceding claims, **characterized in that** the reservoir (200) is a dialyzate mixing device (200).

7. Apparatus in accordance with any one of the preceding claims, **characterized in that** a pump (51) is arranged in the line (K) and is configured to supply a defined volume or a defined volume flow to the secondary side (22) of the filter (20).

8. Apparatus in accordance with any one of the preceding claims, **characterized in that** a pump (50) is arranged in the first circuit, preferably upstream of the primary side (21) of the filter (20) to effect a flow of liquid in the first circuit; and/or **in that** a pressure relief device (60) by means of which pressure can be set on the primary side (21) of the filter (20) is provided in the first circuit, preferably downstream of the filter (20).

9. Apparatus in accordance with any one of the preceding claims, **characterized in that** one or more sensors, preferably one or more conductivity measuring cells (L1, L3) are arranged in the first circuit and are arranged upstream and downstream of the primary side (21) of the filter (20); and/or **characterized in that** one or more sensors, preferably one or more conductivity measuring cells (L2, L4) are arranged in the second circuit in the line (K) and/or in the filtrate line (F).

10. Apparatus in accordance with any one of the preceding claims, **characterized in that** one or more pressure measuring devices (S1, S2) are arranged downstream of the secondary side (22) of the filter (20) and/or upstream of the primary side (21) of the filter (20).

11. Apparatus in accordance with any one of the preceding claims, **characterized in that** the apparatus comprises an ultrafiltrate pump for removing dialysis solution, preferably from the return line (40) from the dialyzer (100) to the container (10) and/or comprises a balancing chamber (B) for the balanced supply and removal of dialysis solution to and from the dialyzer (100).

12. Apparatus in accordance with any one of the preceding claims, **characterized in that** the filter (20) is impermeable to gas; and/or **in that** the second circuit does not have a degassing device.

13. Apparatus in accordance with any one of the preceding claims, **characterized in that** the first and/or second circuits are closed or open.

14. Apparatus in accordance with any one of the preceding claims, **characterized in that** the container (10) is configured as a rigid vessel or as a bag, in particular as a flexible bag, further in particular as a single-use (disposable) article; and/or **characterized in that** the apparatus forms a dialysis machine or a part of a dialysis machine.

15. Method of preparing a dialysis solution using an apparatus in accordance with any one of the claims 1 to 14, **characterized in that** the solution is supplied from the container (10) to the primary side (21) of the filter (20) and the retentate is returned into the container (10), wherein a dialysis solution or a dialysis concentrate is supplied to the secondary side (22) of the filter (20) that is mixed with the permeate of the filter (20) on its secondary side (22).

16. Method in accordance with claim 15, **characterized in that** the permeate is supplied together with the dialysis solution or the dialyzate concentrate to a reservoir (200) in which a ready-to-use dialysis solution is prepared that is supplied to a dialyzer (100).

17. Method in accordance with claim 16, **characterized in that** the mixing region (200) is formed by the reservoir (200).

18. Method in accordance with any one of the claims 15 to 17, **characterized in that** the dialyzate concentrate contains only one single conductive component apart from water.

19. Method in accordance with any one of the claims 15 to 18, **characterized in that** a pump (51) is arranged in the line (K) and supplies a defined volume or a defined volume flow of dialysis solution or of dialyzate concentrate to the secondary side (22) of the filter (20).

20. Method in accordance with any one of the claims 15 to 19, **characterized in that** permeate arising on the secondary side (22) of the filter (20) is conducted together with the supplied dialysis solution or dialyzate concentrate into a balancing chamber (BK).

21. Method in accordance with any one of the claims 15 to 20, **characterized in that** permeate generated on the secondary side (22) of the filter (20) is conducted together with the supplied dialysis solution or dialyzate concentrate into the reservoir (200) or into the mixing region (200).

## Revendications

1. Dispositif pour la production d'une solution de dialyse, le dispositif présentant un premier circuit et un deuxième circuit, le premier circuit présentant un récipient (10) pour la réception de la solution de dialyse utilisée ou d'eau fraîche ou d'un autre liquide, le côté primaire (21) d'un filtre (20) monté en aval du récipient (10) ainsi qu'une conduite de retour (30) du côté primaire (21) du filtre (20) dans le récipient (10), le filtre (20) étant réalisé pour produire de l'eau purifiée à partir de la solution de dialyse utilisée ou à partir d'eau fraîche ou à partir d'un autre liquide, et le deuxième circuit présentant le côté secondaire (22) du filtre (20), le côté dialysat d'un dialyseur (100), un réservoir (200), une conduite (K) menant du réservoir (200) au côté secondaire (22) du filtre, au moyen de laquelle du dialysat ou un concentré de dialysat peut être amené au côté secondaire (22) du filtre (20), ainsi qu'une conduite de filtrat (F), qui s'éloigne du côté secondaire (22) du filtre (20).

2. Dispositif selon la revendication 1, **caractérisé en ce qu'**il existe une conduite de retour (40) du côté dialysat du dialyseur (100) dans le récipient (10) ou **en ce qu'**il existe une conduite (41') du côté dialysat du dialyseur (100) dans une évacuation.

3. Dispositif selon la revendication 1 ou 2, **caractérisé en ce que** le filtre (20) consiste en un filtre en graphène.

4. Dispositif selon l'une quelconque des revendications précédentes, **caractérisé en ce que** la conduite de filtrat (F) mène du côté secondaire (22) du filtre (20) à une chambre d'équilibrage (BK) du dispositif et/ou au réservoir (200).

5. Dispositif selon l'une quelconque des revendications précédentes, **caractérisé en ce que** le réservoir (200) contient une solution de dialyse prête à l'emploi ou un concentré, particulièrement un concentré de bicarbonate, qui est utilisé pour la production d'une solution de dialyse prête à l'emploi.

6. Dispositif selon l'une quelconque des revendications précédentes, **caractérisé en ce que** le réservoir (200) consiste en un dispositif de mélange de dialysat (200).

7. Dispositif selon l'une quelconque des revendications précédentes, **caractérisé en ce qu'**une pompe (51) est agencée dans la conduite (K), qui est réalisée pour amener un volume défini ou un débit défini au côté secondaire (22) du filtre (20).

8. Dispositif selon l'une quelconque des revendications précédentes, **caractérisé en ce qu'**une pompe (50) est agencée dans le premier circuit, de préférence en amont du côté primaire (21) du filtre (20), afin de provoquer un écoulement de liquide dans le premier circuit et/ou **en ce qu'**un appareil de limitation de pression (60) est prévu dans le premier circuit, de préférence en aval du filtre (20), au moyen duquel la pression du côté primaire (21) du filtre (20) peut être réglée.

9. Dispositif selon l'une quelconque des revendications précédentes, **caractérisé en ce que** dans le premier circuit sont agencés un ou plusieurs capteurs, de préférence une ou plusieurs cellules de mesure de conductivité (L1, L3), qui sont agencés en amont et en aval du côté primaire (21) du filtre (20) et/ou **caractérisé en ce que** dans le deuxième circuit sont agencés dans la conduite (K) et/ou dans la conduite de filtrat (F) un ou plusieurs capteurs, de préférence une ou plusieurs cellules de mesure de conductivité (L2, L4).

10. Dispositif selon l'une quelconque des revendications précédentes, **caractérisé en ce qu'**en aval du côté secondaire (22) du filtre (20) et/ou en amont du côté primaire (21) du filtre (20) sont agencés un ou plusieurs appareils de mesure de pression (S1, S2).

11. Dispositif selon l'une quelconque des revendications précédentes, **caractérisé en ce que** le dispositif présente une pompe à ultrafiltrat pour l'évacuation de la solution de dialyse, de préférence depuis la conduite de retour (40) du dialyseur (100) vers le récipient (10) et/ou une chambre d'équilibrage (B) pour l'amenée et l'évacuation équilibrées de la solution de dialyse vers et depuis le dialyseur (100).

12. Dispositif selon l'une quelconque des revendications précédentes, **caractérisé en ce que** le filtre (20) est imperméable aux gaz et/ou **en ce que** le deuxième circuit ne présente pas d'appareil de dégazage.

13. Dispositif selon l'une quelconque des revendications précédentes, **caractérisé en ce que** le premier et/ou le deuxième circuit sont fermés ou ouverts.

14. Dispositif selon l'une quelconque des revendications précédentes, **caractérisé en ce que** le récipient (10) est réalisé sous forme de récipient rigide ou sous forme de poche, particulièrement sous forme de poche flexible, plus particulièrement sous forme d'article à usage unique (jetable) et/ou **caractérisé en ce que** le dispositif forme un appareil de dialyse ou une partie d'un appareil de dialyse.

15. Procédé de production d'une solution de dialyse avec un dispositif selon l'une quelconque des revendications 1 à 14, **caractérisé en ce que** la solution provenant du récipient (10) est amenée au côté primaire (21) du filtre (20) et le rétentat est renvoyé dans le récipient (10), une solution de dialyse ou un concentré de dialysat étant amené au côté secondaire (22) du filtre (20), lequel est mélangé avec le perméat du filtre (20) sur son côté secondaire (22).

16. Procédé selon la revendication 15, **caractérisé en ce que** le perméat est amené, conjointement avec la solution de dialyse ou le concentré de dialysat, à un réservoir (200) dans lequel est produite une solution de dialyse prête à l'emploi qui est amenée à un dialyseur (100),

17. Procédé selon la revendication 16, **caractérisé en ce que** la zone de mélange (200) est formée par le réservoir (200).

18. Procédé selon l'une quelconque des revendications 15 à 17, **caractérisé en ce que** le concentré de dialysat ne contient qu'un seul composant conducteur en plus de l'eau.

19. Procédé selon l'une quelconque des revendications 15 à 18, **caractérisé en ce qu'**une pompe (51) est agencée dans la conduite (K), laquelle amène au côté secondaire (22) du filtre (20) un volume défini ou un débit défini de solution de dialyse ou de concentré de dialysat.

20. Procédé selon l'une quelconque des revendications 15 à 19, **caractérisé en ce que** le perméat formé sur le côté secondaire (22) du filtre (20) est envoyé dans une chambre d'équilibrage (BK) conjointement avec la solution de dialyse ou le concentré de dialysat amené.

21. Procédé selon l'une quelconque des revendications 15 à 20, **caractérisé en ce que** le perméat formé sur le côté secondaire (22) du filtre (20) est envoyé dans le réservoir (200) ou dans la zone de mélange (200) conjointement avec la solution de dialyse ou le concentré de dialysat amené.
